(19) **Europäisches Patentamt**
**European Patent Office**
**Office européen des brevets**

(11) **EP 3 458 992 B1**

(12) **EUROPEAN PATENT SPECIFICATION**

(45) Date of publication and mention
of the grant of the patent:
**02.03.2022 Bulletin 2022/09**

(21) Application number: **16725936.5**

(22) Date of filing: **19.05.2016**

(51) International Patent Classification (IPC):
***G16B 40/00*** *(2019.01)* ***G16B 20/20*** *(2019.01)*

(52) Cooperative Patent Classification (CPC):
**G16B 20/20; G16B 40/00;** G16B 20/00

(86) International application number:
**PCT/IB2016/052936**

(87) International publication number:
**WO 2017/199067 (23.11.2017 Gazette 2017/47)**

(54) **BIOMARKERS SIGNATURE DISCOVERY AND SELECTION**

ENTDECKUNG UND AUSWAHL DER SIGNATUR VON BIOMARKERN

DÉCOUVERTE ET SÉLECTION DE SIGNATURES DE BIOMARQUEURS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**27.03.2019 Bulletin 2019/13**

(73) Proprietor: **Quartz Bio SA
1202 Genève (CH)**

(72) Inventors:
• **BARRETO-SANZ, Miguel
1012 Lausanne (CH)**
• **PEÑA REYES, Carlos Andrés
2824 Vicques (CH)**

(74) Representative: **KATZAROV S.A.
Geneva Business Center
12 Avenue des Morgines
1213 Petit-Lancy (CH)**

(56) References cited:
**US-A1- 2012 040 861 US-A1- 2012 115 138**

• **HABTOM ET AL: "Classification algorithms for
phenotype prediction in genomics and
proteomics", FRONTIERS IN BIOSCIENCE, vol.
13, no. 13, 1 January 2008 (2008-01-01), page 691,
XP055340083, US ISSN: 1093-9946, DOI:
10.2741/2712**

## Description

### Field of the invention

The present invention concerns a method for discovering biomarkers signature, a device, a use and a computer program product related thereof.

### Description of related art

In the biomedical field, there is a constant need to identify biomolecules (proteins, nucleic acids for instance) or physiological parameters called biomarkers, that are indicative of a specific biological status. Biomarkers are not only useful for diagnosis and prognosis of many diseases, but also for understanding the basis for development of therapeutics. Successful and effective identification of biomarkers can accelerate new drug development process.

Recent technologies of genomics and proteomics emergences, including high throughput screening, supplies a wealth of information regarding biomarkers, such as numbers and forms of proteins expressed in a cell. It is possible to identify for each cell, a profile of expressed proteins characteristic of a particular patient status, either sick or healthy status. Additional information are provided by experimental measurements of physiological parameters of the patient for instance blood pressure, weight or cardio/renal related data.

Consequently, comparing biomarkers input from a patient with a disease to that of a healthy patient can provide opportunities to identify a set of biomarkers, called a signature, that are relevant for diagnosing, monitoring, prognosis or predicting a disease. In this respect, several computer-based methods have been developed to identify signatures that best discriminate a sample from a sick patient from the one of a healthy patient.

For instance, the document WO2013190086 teaches a method combining Significance Analysis of Microarrays (SAM) analysis and Limma analysis or Matthew correlation to generate a signature.

Alternatively, the document EP0827611 discloses a method to generate signatures based on fuzzy logic to identify biomarkers chosen amongst cells pools, regulators, chemical production, human or anatomical response and manifestation of the disease at different level or hierarchy.

The document US2012115138 concerns a method for the diagnosis of a disease. The diagnosis is made by measuring at least two different species of biomolecules and classifying the results by means of suitable classifier algorithms and other statistical procedures.

The document US2012040861 provides a list of biomarkers that can be used alone or in various combinations to diagnose cancer generally or pancreatic cancer specifically. The method of US2012040861 is based on a comparison between a biomarker profile from an individual to be tested and a biomarker reference profile of an individual classified as having pancreatic cancer.

The document RESSOM Habtom, Classification algorithms for phenotype prediction in genomics and proteomics, FRONTIERS IN BIOSCIENCE, 2008, vol. 13, n° 13, page 691, relates to a statistical and machine learning-based feature selection and pattern classification algorithms and their application in molecular cancer classification or phenotype prediction.

The document PENA-REYES, Carlos Andres, "Coevolutionary fuzzy modelling" (2002), teaches a method to provide a biomarkers signature with fuzzy logic and genetic algorithm from biomedical data. Fuzzy logic is based on the assumption that a statement may be partially right (or false) in contrast with a Boolean system. Fuzzy logic is particularly suitable for processing biomedical data by allowing a more accurate description of the evolution of a medical status, for instance from a healthy to a sick status. Fuzzy logic permits to take into account the variations and the intermediate levels of a status whereas Boolean system would focus on arbitrary status, either sick or healthy for instance. In the document PENA-REYES, fuzzy logic is combined with genetic algorithm. Genetic algorithm is a well-recognized technique that takes into account the natural evolution of genetic information, in particular the Darwinian principle of survival of the fittest.

Classically, the first step to identify an accurate signature concerns the generation of a set of signatures comprising up to several thousands of signatures, each signature generally reciting several dozen or hundreds of biomarkers. It is necessary to generate several signatures to optimize the chance of identifying at least one accurate signature. Later on, a user analyses and compares the generated signatures among the set to select one or more of them. It is possible to provide satisfying results with a manual method if the set of signatures to be sorted out is limited, typically below a dozen signatures.

However, when it comes to sorting out the most accurate signature among a set of hundreds or thousands, the manual selection process is not adapted any more, mainly because this process is very time consuming. Moreover, the manual selection is error prone when the number of signature to be sorted is important.

Therefore, there is a need for a method to sort out at least a target signature among a set of signature in an efficient and accurate manner.

Brief summary of the invention

One of the aim of the invention is to provide a method for discovering biomarker signature free from, or at least minimizing, the limitations of the known methods.

Another aim of the invention is to provide a method for discovering at least one signature in an accurate and efficient manner in particular when the set of signatures comprises a great number of signatures, for instance above twenty to fifty signatures.

According to the invention, at least a part of these aims are achieved by means of a computed implemented method for discovering at least a biomarker signature from biomarker data pools, said biomarkers signature being designed for being used for diagnosing, predicting or monitoring a disease, the method comprising the steps of:

i) Generating a set of signatures with fuzzy logic and evolutionary algorithms, each signature reciting a determined number of biomarkers;

ii) Selecting at least a target signature from said set of signatures by applying at least the following filters on said set of signatures:

a) a performance filter comprising:

- setting a performance threshold for at least a performance criterion;
- removing signature having a value below said performance threshold for said performance criteria;

b) a frequency filter for sorting said set of signatures depending on the frequency of one biomarker within said set of signatures or the co-frequency of several biomarkers within the same signature among said set of signatures; and

c) a dominance filter comprising:

- ranking the set of signatures depending on at least one performance criteria;
- computing a dominance value for each signature, the dominance value being the number of signatures with a superior ranking for said performance criteria;
- setting a dominance threshold and removing the signatures having a dominance value higher than said dominator threshold.

The method according to the present invention allows sorting out at least a target signature among a set of signatures. The inventors discovered that by applying at least one performance filter, at least one frequency filter and at least one dominance filter to a set of signatures generated by fuzzy logic and genetic algorithm, it is possible to select at least one target signature in a more efficient and accurate manner than with the existing methods.

The present invention allows providing robust target signatures because the target signatures are selected in a soft, fine-tuned and stepwise approach.

In the present invention, the use of at least a performance filter, a frequency filter and a dominance filter have a synergic effect, meaning that the selection of target signatures is more efficient by using at least a performance filter, a frequency filter and a dominance filter than when one of said filters is applied individually on the set of signatures.

Advantageously, the present invention allows minimizing the number of biomarker in the target signature while maintaining exceptional results, for instance in terms of accuracy, sensitivity and specificity. Similarly, the method according to the present invention also permits to minimize the number of rules in the target signature.

By constraining the number of rules and biomarkers in each of the signature, testing costs based on the target signature will be reduced, both on the development end and consumer end. A cleaner, more concise target signature can also aid developers in navigating the regulatory approval process likely to follow the discovery of a target signature.

Performance filter is a filter that sorts out the signatures based on a threshold value of a performance criteria in learning. For instance, the performance criteria is chosen among specificity, sensitivity, accuracy, positive and negative predictive values (PPV and NPV respectively), number of biomarkers or rules per signature, area under the ROC curve (AUC), and average distance measurement (ADM).

Sensitivity is a parameter focusing on sick people by describing the proportion of true positives, i.e. sick people, that are correctly identified as such among those who have the disease. Sensitivity is defined as :

$$\text{Sensitivity: } TruePos / (TruePos + FalseNeg)$$

Specificity is a parameter concerning healthy people by describing the proportion of true negatives, i.e. healthy people, that are correctly identified as such among those whose are healthy. Specificity is defined as :

$$Specificity:\ TrueNeg\ /\ (TrueNeg\ +\ FalsePos)$$

In the present invention, the accuracy is a parameter that takes into account the specificity and the sensitivity , said accuracy being defined as:

$$Accuracy:\ (TruePos\ +TrueNeg)\ /\ (TruePos\ +\ TrueNeg\ +$$
$$FalsePos\ +\ FalseNeg)$$

Positive and negative predictive values (PPV and NPV respectively) concerns the proportions of positive and negative results that are true positive and true negative results. PPV is defined as :

$$PPV:\ TruePos\ /\ (TruePos\ +\ FalsePos)$$

NPV is defined as:

$$NPV:\ TrueNeg\ /\ (TrueNeg\ +\ FalseNeg)$$

In one embodiment, the application of one frequency filter comprises:

- selecting at least one biomarker listed in the set of signatures;

- removing the signature(s) free from said selected biomarker.

For instance, if the frequency filter is set on a biomarker A, then, all the signature comprising the biomarker A will be selected. Similarly, the frequency filter can also be used to select the co-frequency of two or more biomarkers within one signature.
According to an embodiment, the application of one frequency filter comprises:

- computing a frequency of each biomarker in the set of signatures;

- defining a frequency threshold for at least one biomarker;

- removing the signature(s) comprising biomarker(s) with a frequency below said frequency threshold.

For instance, in this embodiment the frequency filter allows removing the signatures comprising biomarkers little used in the set of signatures.
The inventors found out that the dominance filter is an efficient filter to provide accurate target signatures. The dominance filter is used to compare the signatures depending on at least one performance criteria. First, the signatures are ranked depending on at least a performance criteria, said performance criteria can be the same than the performance criteria used in the performance filter or a different one. Then, a dominance threshold is set and the signature are sorted out by comparing their respective dominance value with the dominance threshold: if a signature has a dominance value above the dominance threshold, said signature is removed.
For instance, a dominance filter uses the sensitivity and the specificity as performance criteria. The set of signatures to be sorted out is the following:

- Signature A: specificity 80%, sensitivity 90%

- Signature B: specificity 90%, sensitivity 80%

- Signature C: specificity 60%, sensitivity 60%

In the present example, the dominance values are the following:

- Signature A: 1 (dominated by B in specificity)

- Signature B: 1 (dominated by A in sensitivity )

- Signature C: 2 (dominated by A and B in specificity; dominated by A and B in sensitivity )

In the present case, the signature C is always dominated by two signatures, either in sensitivity or in specificity. A dominance threshold is set to two, meaning the all the signatures being dominated by two or more signatures, called dominators, is removed. Thus, signature C is removed from the set of signatures.

The dominance filter allows sorting out the set of signatures by comparing signatures to each other's: a signature is selected if said signature dominates "X" other signatures ("X" being the dominance threshold). On the contrary, with the performance filter for instance, a signature is selected if said signature has a performance value above a threshold. The advantage of the dominance compared with other filters is that it allows to select several good alternatives. Each option is first assessed under multiple criteria and then a subset of options is identified with the property that no other option can categorically outperform any of its members. By yielding all of the potentially optimal solutions, the selection can make focused trade-offs within this constrained set of parameters, rather than needing to consider the full ranges of parameters.

In one embodiment, the performance criteria of the dominance filter are the specificity and the sensitivity.

In one embodiment, the performance criteria of the dominance filter is the specificity.

In one embodiment, the performance criteria of the dominance filter is the sensitivity.

In one embodiment, the performance criteria of the dominance filter is the accuracy.

In another embodiment, the performance criteria used in the performance filter are the sensitivity and the specificity.

In one embodiment, the target signature is selected by using successively at least one performance filter, at least one frequency filter and at least one dominance filter.

According to an embodiment, step ii) comprises:

- applying several sequence of filters on the set of signatures, each filter sequence comprising at least one filter, each filter sequence being applied separately on the set of signatures so that each filter sequence provides at least one preselection of signature;

- identifying at least one common signature, said common signature being at least one signature present in all the preselection;

- combining the common signature(s);

- applying at least one filter on the common signature(s).

A sequence of filters comprises at least one filter. In this embodiment, several sequences of filters are applied separately, i.e. in parallel, meaning that each sequence of filter is applied on the set of signature to provide one preselection of signature. Each preselection comprises a determined number of signatures. When one specific signature is listed in several preselections, said signature is designated as a common signature. The common signatures are combined and subsequently filtered.

In one embodiment, step ii) comprises the successive steps of:

- applying a first performance filter on the set of signatures;

- applying at first frequency filter on the signature isolated in the previous step to provide a first preselection of signature;

- applying a first dominance filter independently on the signature isolated by the first performance filter to provide a second preselection of signature;

- combining the signature listed in both the first preselection and the second preselection;

- applying a second dominance filter on the signature isolated in the previous step;

- applying a second performance filter on the signature isolated in the previous step;

- applying a third performance filter on the signature isolated in the previous step;

- applying an expert filter on the signature isolated in the previous step.

In this embodiment, two sequence filters are applied on the set of signatures, a first filter sequence comprising at least a frequency filter providing a first preselection and a second filter sequence comprising at least a dominance filter providing a second preselection. Signatures selected in both the first preselection and the second preselection are combined and filtered subsequently. The inventors found out that this embodiment provides reliable target signatures, because each target signature is selected by two independent sequence filters.

In one embodiment, several iterations of the method are performed, each iterations providing a family of target signatures. A family of target signatures can be provided to meet the specific needs of a client, for instance, some families are focused on sensitivity, others have a limited number of biomarkers. A family of target signatures gathers at least two target signatures with a special feature. A family of target signature can be generated by one iteration of the method according to the invention. Several families of target signature can be generated by running several iteration of the method according to the present invention.

In one embodiment, the target signature(s) comprises at least one rule. The rule(s) permits to define the relationship between the biomarkers.

According to an embodiment, the method further comprises an expert filter applied by an expert in signature discovery to remove at least one biomarker from the target signature. For instance, the expert filter is used as a last step of the method, to fine tune the target signature. For instance, the expert filter is used to remove at least an irrelevant variable that remains after artificial evolution. The expert filter can also be used to choose in favour of a defined biomarker to meet a client request.

According to an embodiment, the data pools comprises data chosen amongst protein or nucleic acid measurements, physiological parameters such as age, weight, gender, or other clinical data. For instance, the data pools comprise plasma/blood concentrations of biomolecules, or measurements of physiological parameters of healthy and sick patients.

In one embodiment, the data pools comprise biomedical data from sick and from healthy patients. In particular the data pools comprise biomedical data from sick and from healthy human patients. The data pools can also comprise biomedical data from patients developing certain disease. The data pools can also comprise biomedical data from patients developing from patients at different disease stages. One or several pool(s) can comprise data from healthy patients and (an) other(s) pool(s) can comprise data from sick patient.

In one embodiment, the data pools comprises data from sick and from healthy plants. Thus, the method according to the present invention can be used with plants to discover biomarker signature comprising plants' biomarkers. The data pools from plants can comprise data from any healthy or sick plant, in particular crops for food production human or animal (for instance corn, soya etc), textile production (including cotton, Corchorus genus, Linum usitatissimum etc), plants used to create biofuels (including wheat, corn, sugar beets, sugar cane etc), medicinal plants (aloe vera, Wild Ginger, Belladonna etc), for production of alcoholic beverages (including vineyard, blue agave etc).

In one embodiment, the data pools comprises data from sick and from healthy animal. Thus, the method according to the present invention can be used with animals to discover biomarker signature comprising animals' biomarkers. The data pools from animals can comprise data from any healthy or sick animals, domestic animals or wild animals. In particular, animals are used for food production (chickens, fish, bees, cows), domestic animals (cats, dogs etc), animal in sport (including horses, dogs etc) and animals from pre-clinical studies.

Another aim of the invention is to provide a device for discovering at least a biomarkers signature from biomarker data pools free from the limitations of the known device.

According to the invention, this aim is achieved by means of a device for discovering at least a biomarkers signature from biomarkers data pools, the device comprising:

- A system combining fuzzy logic with evolutionary algorithms for generating a set of signatures, each signature reciting a determined number of biomarker;

- A filter system for selecting at least a target signature from said set of signatures, said filter system comprising at least the following filters:

  o a performance filter for sorting said set of signatures depending on at least one performance criteria;

  o a frequency filter for sorting said set of signatures depending on the frequency of one biomarker within said set of signatures or the co-frequency of several biomarkers within the same signature among said set of signatures;

o a dominance filter, each signature has a dominance value so that the dominance filter is capable of sorting said set of signatures depending on the dominance value.

The invention further concerns a biomarker signature discovered by a method according to the present invention for diagnosing, predicting or monitoring a disease.

In one embodiment, the disease is chosen among plant disease, human disease, animal disease.

The embodiments regarding the method according to the present invention apply mutatis mutandis to the device according to the present invention, and vice versa.

A method or a device according to the present invention can comprise an isolated embodiment.

A method or a device according to the present invention can comprise a combination of a plurality of embodiments.

In the context of the invention, the terms "biomarker" is defined as a characteristic that is objectively measured and evaluated as an indicator of normal biologic processes, pathogenic processes, or pharmacologic responses to a therapeutic intervention, as defined by the National Health Institute (NIH, USA). A biomarker can be a biomolecule, such as protein or nucleic acids, or a physiological parameter (blood pressure for human or animal) of a human, an animal or a plant.

In the context of the invention, the terms "signature" or "biomarker signature" are interchangeable and synonym. The terms "signature" or "biomarker signature" refer to at least two biomarkers that are relevant to describe a particular status of a patient.

In the context of the invention, a target signature can comprise several rules. The term "rule" describes the variation of the biomarker of a signature. In one embodiment, the target signature(s) comprises at least one rule.

For instance, if the signature comprises three biomarkers (BM), namely BM1, BM2, BM3, rules 1 and 2 teach that:

Rule 1: if (BM1 is Low) and (BM2 is Low) and (BM3 is Low) then (the patient is sick)

Rule 2: if (BM1 is high) and (BM2 is Low) and (BM3 is high) then (the patient is sick)

The terms high and low refers for instance to the plasma or blood concentration of biomarker with respect to one or several threshold(s) when the biomarkers are a biomolecule.

On the context of the invention, the term "filter" refers to a mathematical operation allowing to remove at least one signature from the set of signatures.

Brief Description of the Drawings

The invention will be better understood with the aid of the description of two embodiments given by way of examples and illustrated by the figures, in which:

Fig. 1 shows the filters used in a first and second embodiment of the present invention;

Fig. 2 and 3 illustrate the first embodiment the present invention focusing on a colon cancer study;

Fig. 4 and 5 illustrate the second embodiment the present invention focusing on a prostate cancer study;

Detailed Description of possible embodiments of the Invention

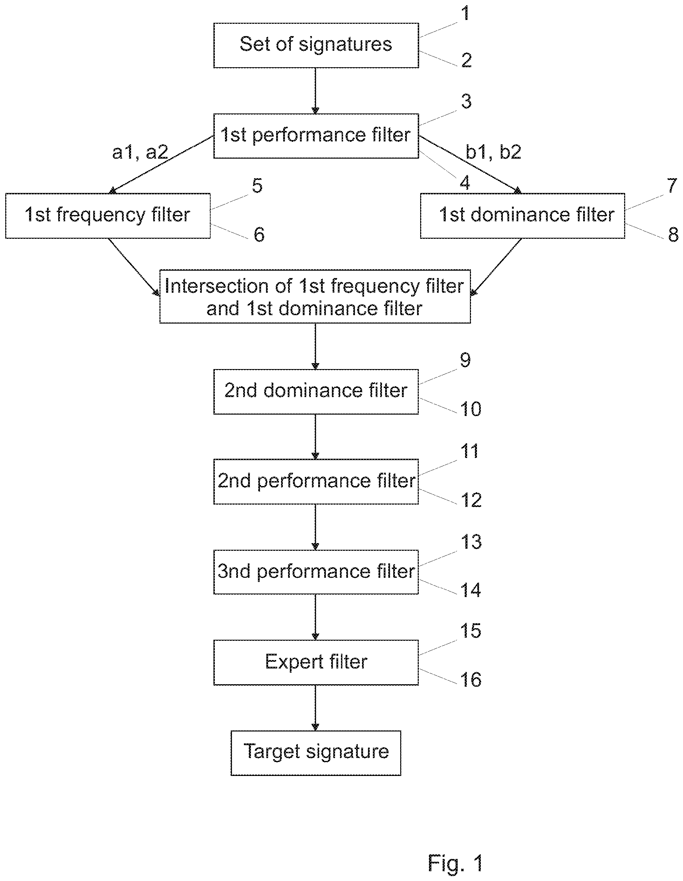

Figure 1 illustrates the filters used in a first and second embodiment of the present invention concerning respectively signatures for human colon cancer diagnosis and human prostate cancer diagnosis but it is intended that the invention is not limited to human disease, the invention can also be applied to plant or animal disease by using respectively plant or animal biomarker database pools.

In the first and second embodiment, the method for signature discovery starts with a first performance filter 3, 4. Then, the method comprises two sequences of filter:

- a first sequence filter a1, a2 comprising a first frequency filter 5, 6;

- a second sequence filter b1, b2 comprising a first dominance filter 7, 8;

Then, the signatures selected both in the first sequence filter a1, a2 and in the second sequence filter b1, b2 are combined and a second dominance filter 9, 10 is applied. Subsequently, a second performance filter 11, 12 and a third performance filter 13, 14 are applied successively. Eventually, an expert filter 15, 16 allows providing the target signature.

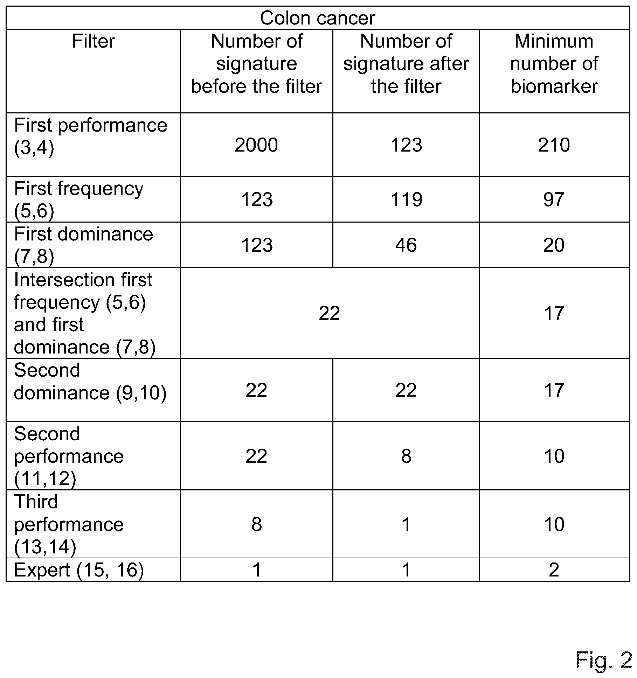

The first embodiment aims at discovery a colon cancer target signature. To that end, a data pool of 40 tumors samples and 22 normal samples is analysed, each sample comprising 6000 genes (Alon, U., Barkai, N., Notterman, D. A., Gish, K., Ybarra, S., Mack, D., & Levine, A. J. (1999). Broad patterns of gene expression revealed by clustering analysis of tumor and normal colon tissues probed by oligonucleotide arrays. Proceedings of the National Academy of Sciences, 96(12), 6745-6750.). A set of 2000 signatures is then generated by using fuzzy logic and genetic algorithm. The set of signatures is sorted out by using the filters illustrated in figure 1 (references 1, 3, a1, 5, b1, 7, 9, 11, 13, 15 of figure 1). The results obtained in this first embodiment are showed in Figure 2. The method according to the present invention allows selecting a target signature comprising 2 biomarkers starting from a set of 2000 signatures. The method also permits to decrease drastically the number of biomarkers in the signature from 210 biomarkers to 2 biomarkers for the target signature.

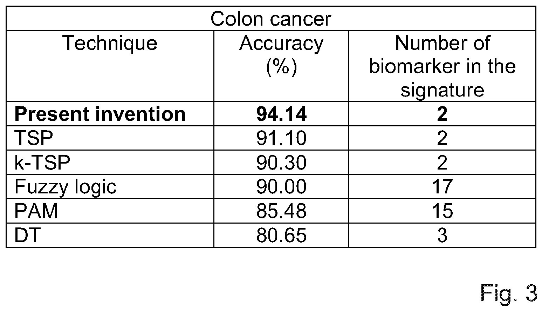

The accuracy of the target signature in the diagnosis of colon cancer was compared to the accuracy provided by other existing techniques, as shown in figure 3. The present invention proved to be the best method with an accuracy of 94.14 % by using a target signature with only 2 biomarkers.

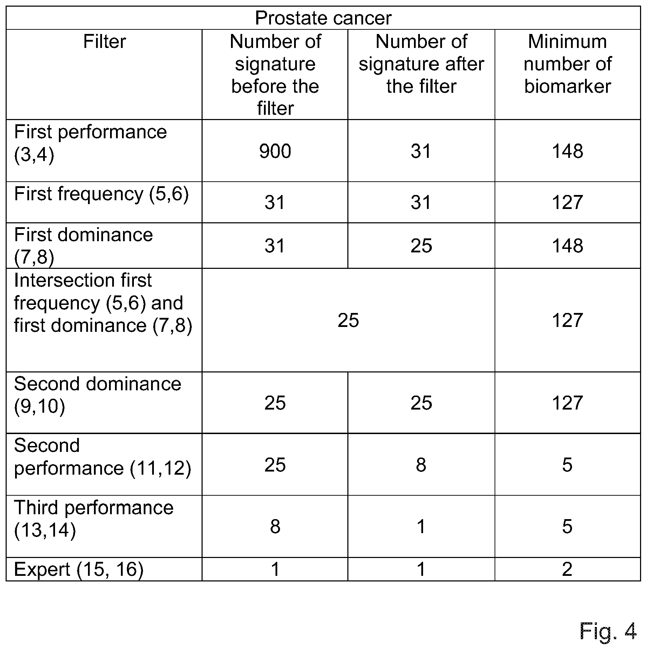

The second embodiment aims at discovering a prostate cancer target signature. To that end, a data pools of 52 tumors samples and 50 normal sample were analysed, each samples comprising 12'600 genes (Singh, D., Febbo, P. G., Ross, K., Jackson, D. G., Manola, J., Ladd, C., ... & Sellers, W. R. (2002). Gene expression correlates of clinical prostate cancer behavior. Cancer cell, 1(2), 203-209). A set of 900 signatures is generated by using fuzzy logic and genetic algorithms. The set of signatures is sorted out by using the filters illustrated in Figure 1 (references 2, 4, a2, b2, 6, 8, 10, 12, 14, 16 of figure 1). The results obtained in this first embodiment are showed in Figure 4. The method according to the present invention allows selecting a target signature comprising 2 biomarkers starting from a set of 900 signatures. The method also permits to decrease drastically the number of biomarkers in the signature from 148 biomarkers to 2 biomarkers for the target signature.

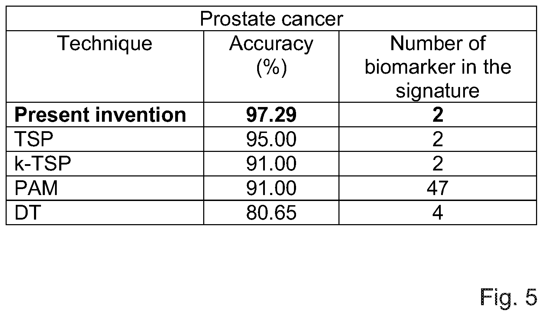

Similarly to the first embodiment, the accuracy of the target signature in the diagnosis of prostate cancer was compared to the accuracy provided by other existing techniques, as shown in figure 5. The present invention proved to be the best method with an accuracy of 97.29 % by using a target signature with only 2 biomarkers.

The details of the acronyms listed in the Figure 3 and 5 are: TSP (Top scoring pair), K-TSP ( k- Top scoring pair), PAM (Prediction analysis of microarrays), DT (C4.5 decision trees).

The invention is also related to a computer program product comprising computer code arranged to be executed by processing means in order to carry out some or all of the above described methods when the processing means execute this computer code.

## Claims

1. Computer implemented method for discovering at least a biomarkers signature from biomarker data pools, said biomarkers signature being designed for being used for diagnosing, predicting or monitoring a disease, the method comprising the steps of:

   i) Generating a set of signatures (1, 2) with fuzzy logic and evolutionary algorithms, each signature reciting a determined number of biomarkers;

   ii) Selecting at least a target signature from said set of signatures (1, 2) by applying at least the following filters on said set of signatures (1, 2):

   a) a performance filter comprising:

   - setting a performance threshold for at least a performance criterion;
   - removing signatures having a value below said performance threshold for said performance criteria;

   b) a frequency filter for sorting said set of signatures depending on the frequency of one biomarker within said set of signatures (1, 2) or the co-frequency of several biomarkers within the same signature among said set of signatures (1, 2); and
   c) a dominance filter comprising:

   - ranking the set of signatures (1, 2) depending on at least one performance criteria;

- computing a dominance value for each signature, the dominance value being the number of signatures with a superior ranking for said performance criteria;
- setting a dominance threshold and removing the signatures having a dominance value higher than said dominator threshold.

2. A method according to claim 1 wherein the target signature is selected by using successively at least one performance filter, at least one frequency filter and at least one dominance filter.

3. A method according to claim 1 wherein step ii) comprises:

- applying several sequence of filters on the set of signatures (1, 2), each filter sequence comprising at least one filter, each filter sequence being applied separately on the set of signatures (1, 2) so that each filter sequence provides at least one preselection of signature;
- identifying at least one common signature, said common signature being at least one signature present in all the preselection;
- combining the common signature(s);
- applying at least one filter on the common signature(s).

4. A method according to claim 1 wherein step ii) comprises the successive steps of:

- applying a first performance filter (3, 4) on the set of signatures (1, 2);
- applying at first frequency filter (5, 6) on the signature isolated in the previous step to provide a first preselection of signature;
- applying a first dominance filter (7, 8) independently on the signature isolated by the first performance filter (3, 4) to provide a second preselection of signature;
- combining the signature listed in both the first preselection and the second preselection;
- applying a second dominance filter (9, 10) on the signature isolated in the previous step;
- applying a second performance filter (11, 12) on the signature isolated in the previous step;
- applying a third performance filter (13, 14) on the signature isolated in the previous step;
- applying an expert filter (15, 16) on the signature isolated in the previous step.

5. A method according to any one of the previous claims wherein the target signature(s) comprises at least one rule.

6. A method according to any one of the previous claims wherein the performance criteria is chosen among specificity, sensitivity , accuracy, positive and negative predictive values (PPV and NPV respectively), number of biomarker or rule per signature, area under the ROC curve (AUC), and average distance measurement (ADM).

7. A method according to any one of the previous claim further comprising an expert filter (15, 16) applied by an expert in signature discovery to remove at least one biomarker from the target signature.

8. A method according to any one of the previous claims wherein the application of one frequency filter comprises:

- selecting at least one biomarker listed in the set of signatures (1, 2);
- removing the signature free from said selected biomarker.

9. A method according to any one of the previous claims wherein the application of one frequency filter comprises:

- computing a frequency of each biomarker in the set of signatures (1, 2);
- defining a frequency threshold for at least one biomarker;
- removing the signature(s) comprising biomarker(s) with a frequency below said frequency threshold.

10. A method according to any one of the previous claims wherein several iterations of the method are performed, each iteration providing a family of target signatures.

11. A method according to any one of the previous claims wherein the data pools comprises biomedical data from sick and from healthy patients.

12. A method according to any one of claims 1 to 10 wherein the data pools comprises data from either sick and from healthy plants, or data from sick and from healthy animals.

13. A method according to any one of the previous claims wherein the data pools comprises data chosen amongst protein or nucleic acid measurements, physiological parameters such as age, weight, gender, or any clinical data.

14. A device for discovering at least a biomarkers signature from biomarker data pools, said biomarkers signature being designed for being used for diagnosing, predicting or monitoring a disease, the device comprising:

- A system combining fuzzy logic with evolutionary algorithms for generating a set of signatures (1, 2), each signature reciting a determined number of biomarker;
- A filter system for selecting at least a target signature from said set of signatures (1, 2), said filter system comprising at least the following filters:

o a performance filter for sorting said set of signatures depending on at least one performance criteria, the performance filter being arranged for setting a performance threshold for at least a performance criterion, and removing signatures having a value below said performance threshold for said performance criteria;
o a frequency filter for sorting said set of signatures depending on the frequency of one biomarker within said set of signatures (1, 2) or the co-frequency of several biomarkers within the same signature among said set of signatures (1, 2) ;
o a dominance filter, each signature has a dominance value so that the dominance filter is capable of sorting said set of signatures (1, 2) depending on the dominance value, the dominance filter being arranged for ranking the set of signatures (1, 2) depending on at least one performance criteria, and computing a dominance value for each signature, the dominance value being the number of signatures with a superior ranking for said performance criteria, and setting a dominance threshold and removing the signatures having a dominance value higher than said dominator threshold.

15. A computer program product comprising computer code executable by processing means in order to carry out the method of any of the claims 1 to 13 when the computer code is executed.

**Patentansprüche**

1. Computerimplementiertes Verfahren zum Entdecken mindestens einer Signatur von Biomarkern aus Biomarker-Datenbeständen, wobei die Signatur von Biomarkern dazu bestimmt ist, zum Diagnostizieren, Vorhersagen oder Überwachen einer Krankheit verwendet zu werden, wobei das Verfahren die folgenden Schritte umfasst:

i) Generieren eines Satzes Signaturen (1, 2) mit Fuzzy-Logik und evolutionären Algorithmen, wobei jede Signatur eine bestimmte Anzahl von Biomarkern rezitiert;
ii) Auswählen mindestens einer Zielsignatur aus dem Satz Signaturen (1, 2) durch Anwenden mindestens der folgenden Filter auf den Satz Signaturen (1, 2):

a) eines Leistungsfilters, umfassend:

- Festlegen einer Leistungsschwelle für mindestens ein Leistungskriterium;
- Entfernen von Signaturen, die einen Wert aufweisen, der unter der Leistungsschwelle für die Leistungskriterien liegt;

b) eines Häufigkeitsfilters zum Sortieren des Satzes Signaturen in Abhängigkeit von der Häufigkeit eines Biomarkers innerhalb des Satzes Signaturen (1, 2) oder der gemeinsamen Häufigkeit mehrerer Biomarker innerhalb derselben Signatur in dem Satz Signaturen (1, 2); und
c) eines Dominanzfilters, umfassend:

- Ordnen des Satzes Signaturen (1, 2) in eine Rangreihe in Abhängigkeit von mindestens einem Leistungskriterium;
- Berechnen eines Dominanzwerts für jede Signatur, wobei der Dominanzwert die Anzahl der Signaturen mit einer höheren Rangordnung für die Leistungskriterien ist;
- Festlegen eines Dominanzschwellenwerts und Entfernen der Signaturen, deren Dominanzwert höher als der Dominatorschwellenwert ist.

2. Verfahren nach Anspruch 1, wobei die Zielsignatur ausgewählt wird, indem nacheinander mindestens ein Leistungsfilter, mindestens ein Häufigkeitsfilter und mindestens ein Dominanzfilter verwendet werden.

3. Verfahren nach Anspruch 1, wobei Schritt ii) Folgendes umfasst:

   - Anwenden mehrerer Sequenzen von Filtern auf den Satz Signaturen (1, 2), wobei jede Filtersequenz mindestens ein Filter umfasst und jede Filtersequenz separat auf den Satz Signaturen (1, 2) angewandt wird, sodass jede Filtersequenz mindestens eine Signaturvorauswahl liefert;
   - Identifizieren mindestens einer gemeinsamen Signatur, wobei die gemeinsame Signatur mindestens eine überall in der Vorauswahl vorhandene Signatur ist;
   - Kombinieren der gemeinsamen Signatur(en);
   - Anwenden mindestens eines Filters auf die gemeinsame(n) Signatur(en).

4. Verfahren nach Anspruch 1, wobei Schritt ii) die folgenden aufeinanderfolgenden Schritte umfasst:

   - Anwenden eines ersten Leistungsfilters (3, 4) auf den Satz Signaturen (1, 2);
   - Anwenden eines ersten Häufigkeitsfilters (5, 6) auf die im vorhergehenden Schritt isolierte Signatur, um eine erste Signaturvorauswahl zu treffen;
   - Anwenden eines ersten Dominanzfilters (7, 8) unabhängig von der mittels des ersten Leistungsfilters (3, 4) isolierten Signatur, um eine zweite Signaturvorauswahl zu treffen;
   - Kombinieren der sowohl in der ersten Vorauswahl als auch in der zweiten Vorauswahl enthaltenen Signatur(en);
   - Anwenden eines zweiten Dominanzfilters (9, 10) auf die im vorhergehenden Schritt isolierte Signatur;
   - Anwenden eines zweiten Leistungsfilters (11, 12) auf die im vorhergehenden Schritt isolierte Signatur;
   - Anwenden eines dritten Leistungsfilters (13, 14) auf die im vorhergehenden Schritt isolierte Signatur;
   - Anwenden eines Expertenfilters (15, 16) auf die im vorhergehenden Schritt isolierte Signatur.

5. Verfahren nach einem der vorhergehenden Ansprüche, wobei die Zielsignatur(en) mindestens eine Regel umfasst (umfassen).

6. Verfahren nach einem der vorhergehenden Ansprüche, wobei die Leistungskriterien aus Spezifität, Sensitivität, Genauigkeit, positivem und negativem Vorhersagewert (PPV bzw. NPV), Anzahl der Biomarker oder Regeln je Signatur, Fläche unter der ROC-Kurve (AUG, en: area under the ROC curve) und mittlerem Distanzmaß (ADM, en: average distance measurement) ausgewählt werden.

7. Verfahren nach einem der vorhergehenden Ansprüche, ferner ein Expertenfilter (15, 16) umfassend, das von einem Experten für die Signaturentdeckung angewandt wird, um mindestens einen Biomarker aus der Zielsignatur zu entfernen.

8. Verfahren nach einem der vorhergehenden Ansprüche, wobei die Anwendung eines Häufigkeitsfilters Folgendes umfasst:

   - Auswählen mindestens eines Biomarkers, der in dem Satz Signaturen (1, 2) enthalten ist;
   - Entfernen der Signatur, die den ausgewählten Biomarker nicht aufweist.

9. Verfahren nach einem der vorhergehenden Ansprüche, wobei die Anwendung eines Häufigkeitsfilters Folgendes umfasst:

   - Berechnen der Häufigkeit jedes Biomarkers in dem Satz Signaturen (1, 2);
   - Definieren einer Häufigkeitsschwelle für mindestens einen Biomarker;
   - Entfernen der Signatur(en), die Biomarker mit einer Häufigkeit unter der Häufigkeitsschwelle umfasst (umfassen).

10. Verfahren nach einem der vorhergehenden Ansprüche, wobei mehrere Iterationen des Verfahrens ausgeführt werden, wobei jede Iteration eine Familie von Zielsignaturen liefert.

11. Verfahren nach einem der vorhergehenden Ansprüche, wobei die Datenbestände biomedizinische Daten von kranken und von gesunden Patienten umfassen.

12. Verfahren nach einem der Ansprüche 1 bis 10, wobei die Datenbestände entweder Daten von kranken und von gesunden Pflanzen oder Daten von kranken und von gesunden Tieren umfassen.

13. Verfahren nach einem der vorhergehenden Ansprüche, wobei die Datenbestände Daten umfassen, die aus Protein- oder Nukleinsäuremessungen, physiologischen Parametern wie Alter, Gewicht, Geschlecht oder klinischen Daten ausgewählt werden.

14. Vorrichtung zum Entdecken mindestens einer Signatur von Biomarkern aus Biomarker-Datenbeständen, wobei die Signatur von Biomarkern dazu bestimmt ist, zum Diagnostizieren, Vorhersagen oder Überwachen einer Krankheit verwendet zu werden, wobei die Vorrichtung Folgendes umfasst:

  - ein System, das Fuzzy-Logik mit evolutionären Algorithmen kombiniert, um einen Satz Signaturen (1, 2) zu generieren, wobei jede Signatur eine bestimmte Anzahl von Biomarkern rezitiert;
  - ein Filtersystem zum Auswählen mindestens einer Zielsignatur aus dem Satz Signaturen (1, 2), wobei das Filtersystem mindestens die folgenden Filter umfasst:

    o ein Leistungsfilter zum Sortieren des Satzes Signaturen in Abhängigkeit von mindestens einem Leistungskriterium, wobei das Leistungsfilter derart eingerichtet ist, dass ein Leistungsschwellenwert für mindestens ein Leistungskriterium festgelegt wird und Signaturen, die einen Wert aufweisen, der unter dem Leistungsschwellenwert für die Leistungskriterien liegt, entfernt werden;
    o ein Häufigkeitsfilter zum Sortieren des Satzes Signaturen in Abhängigkeit von der Häufigkeit eines Biomarkers innerhalb des Satzes Signaturen (1, 2) oder der gemeinsamen Häufigkeit mehrerer Biomarker innerhalb derselben Signatur in dem Satz Signaturen (1, 2);
    o ein Dominanzfilter, wobei jede Signatur einen Dominanzwert aufweist, sodass das Dominanzfilter imstande ist, den Satz Signaturen (1, 2) in Abhängigkeit von dem Dominanzwert zu sortieren, wobei das Dominanzfilter dazu eingerichtet ist, dass es den Satz Signaturen (1, 2) in Abhängigkeit von mindestens einem Leistungskriterium in eine Rangreihe ordnet und für jede Signatur einen Dominanzwert berechnet, wobei der Dominanzwert die Anzahl der Signaturen mit einer höheren Rangordnung für die Leistungskriterien ist, und dass es einen Dominanzschwellenwert festlegt und die Signaturen, deren Dominanzwert höher als der Dominatorschwellenwert ist, entfernt.

15. Computerprogrammprodukt, das Computercode umfasst, der mittels Verarbeitungsmitteln ausführbar ist, um das Verfahren nach einem der Ansprüche 1 bis 13 durchzuführen, wenn der Computercode ausgeführt wird.

**Revendications**

1. Procédé mis en œuvre par ordinateur pour découvrir au moins une signature de biomarqueurs à partir de groupes de données de biomarqueurs, ladite signature de biomarqueurs étant conçue pour être utilisée pour le diagnostic, la prédiction ou la surveillance d'une maladie, le procédé comprenant les étapes de :

  i) génération d'un ensemble de signatures (1, 2) avec une logique floue et des algorithmes évolutifs, chaque signature représentant un nombre déterminé de biomarqueurs ;
  ii) sélection d'au moins une signature cible à partir dudit ensemble de signatures (1, 2) en appliquant au moins les filtres suivants sur ledit ensemble de signatures (1, 2) :

    a) un filtre de performance comprenant :

      - la fixation d'un seuil de performance pour au moins un critère de performance ;
      - la suppression des signatures ayant une valeur en dessous dudit seuil de performance pour ledit critère de performance ;

    b) un filtre de fréquence pour trier ledit ensemble de signatures en fonction de la fréquence d'un biomarqueur dans ledit ensemble de signatures (1, 2) ou de la co-fréquence de plusieurs biomarqueurs dans la même signature parmi ledit ensemble de signatures (1, 2) ; et
    c) un filtre de dominance comprenant :

      - le classement de l'ensemble de signatures (1, 2) en fonction d'au moins un critère de performance ;

- le calcul d'une valeur de dominance pour chaque signature, la valeur de dominance étant le nombre de signatures avec un classement supérieur pour ledit critère de performance ;
- la fixation d'un seuil de dominance et la suppression des signatures ayant une valeur de dominance supérieure audit seuil de dominance.

**2.** Procédé selon la revendication 1 dans lequel la signature cible est sélectionnée en utilisant successivement au moins un filtre de performance, au moins un filtre de fréquence et au moins un filtre de dominance.

**3.** Procédé selon la revendication 1 dans lequel l'étape ii) comprend :

- l'application de plusieurs séquences de filtres sur l'ensemble de signatures (1, 2), chaque séquence de filtres comprenant au moins un filtre, chaque séquence de filtres étant appliquée séparément sur l'ensemble de signatures (1, 2) de sorte que chaque séquence de filtres fournisse au moins une présélection de signature ;
- l'identification d'au moins une signature commune, ladite signature commune étant au moins une signature présente dans toutes les présélections ;
- la combinaison de la/des signature(s) commune(s) ;
- l'application d'au moins un filtre sur la/les signature(s) commune(s).

**4.** Procédé selon la revendication 1 dans lequel l'étape ii) comprend les étapes successives de :

- application d'un premier filtre de performance (3, 4) sur l'ensemble de signatures (1, 2) ;
- application d'un premier filtre de fréquence (5, 6) sur la signature isolée dans l'étape précédente pour fournir une première présélection de signature ;
- application d'un premier filtre de dominance (7, 8) indépendamment sur la signature isolée par le premier filtre de performance (3, 4) pour fournir une deuxième présélection de signature ;
- combinaison des signatures énumérées à la fois dans la première présélection et la deuxième présélection ;
- application d'un deuxième filtre de dominance (9, 10) sur la signature isolée dans l'étape précédente ;
- application d'un deuxième filtre de performance (11, 12) sur la signature isolée dans l'étape précédente ;
- application d'un troisième filtre de performance (13, 14) sur la signature isolée dans l'étape précédente ;
- application d'un filtre expert (15, 16) sur la signature isolée dans l'étape précédente.

**5.** Procédé selon l'une quelconque des revendications précédentes dans lequel la/les signature(s) cible(s) comprend/comprennent au moins une règle.

**6.** Procédé selon l'une quelconque des revendications précédentes dans lequel les critères de performance sont choisis parmi la spécificité, la sensibilité, la précision, les valeurs prédictives positive et négative (PPV et NPV respectivement), le nombre de biomarqueurs ou de règles par signature, l'aire sous la courbe ROC (AUC), et la mesure de distance moyenne (ADM).

**7.** Procédé selon l'une quelconque des revendications précédentes comprenant en outre un filtre expert (15, 16) appliqué par un expert en découverte de signature pour supprimer au moins un biomarqueur de la signature cible.

**8.** Procédé selon l'une quelconque des revendications précédentes dans lequel l'application d'un filtre de fréquence comprend :

- la sélection d'au moins un biomarqueur énuméré dans l'ensemble de signatures (1, 2) ;
- la suppression de la signature exempte dudit biomarqueur sélectionné.

**9.** Procédé selon l'une quelconque des revendications précédentes dans lequel l'application d'un filtre de fréquence comprend :

- le calcul d'une fréquence de chaque biomarqueur dans l'ensemble de signatures (1, 2) ;
- la définition d'un seuil de fréquence pour au moins un biomarqueur ;
- la suppression de la/des signature(s) comprenant un/des biomarqueur(s) ayant une fréquence en dessous dudit seuil de fréquence.

**10.** Procédé selon l'une quelconque des revendications précédentes dans lequel plusieurs itérations du procédé sont effectuées, chaque itération fournissant une famille de signatures cibles.

**11.** Procédé selon l'une quelconque des revendications précédentes dans lequel les groupes de données comprennent des données biomédicales de patients malades ou sains.

**12.** Procédé selon l'une quelconque des revendications 1 à 10 dans lequel les groupes de données comprennent soit des données de plantes malades et saines, soit des données d'animaux malades et sains.

**13.** Procédé selon l'une quelconque des revendications précédentes dans lequel les groupes de données comprennent des données choisies parmi des mesures de protéines ou d'acides nucléiques, des paramètres physiologiques tels que l'âge, le poids, le sexe ou des données cliniques quelconques.

**14.** Dispositif de découverte d'au moins une signature de biomarqueurs à partir de groupes de données de biomarqueurs, ladite signature de biomarqueurs étant conçue pour être utilisée pour le diagnostic, la prédiction ou la surveillance d'une maladie, le dispositif comprenant :

- un système combinant une logique floue avec des algorithmes évolutifs pour générer un ensemble de signatures (1, 2), chaque signature représentant un nombre déterminé de biomarqueurs ;
- un système de filtre pour sélectionner au moins une signature cible à partir dudit ensemble de signatures (1, 2), ledit système de filtre comprenant au moins les filtres suivants :

   o un filtre de performance pour trier ledit ensemble de signatures en fonction d'au moins un critère de performance, le filtre de performance étant agencé pour fixer un seuil de performance pour au moins un critère de performance, et supprimer des signatures ayant une valeur en dessous dudit seuil de performance pour ledit critère de performance,
   o un filtre de fréquence pour trier ledit ensemble de signatures en fonction de la fréquence d'un biomarqueur dans ledit ensemble de signatures (1, 2) ou de la co-fréquence de plusieurs biomarqueurs dans la même signature parmi ledit ensemble de signatures (1, 2) ;
   o un filtre de dominance, chaque signature a une valeur de dominance de sorte que le filtre de dominance soit capable de trier ledit ensemble de signatures (1, 2) en fonction de la valeur de dominance, le filtre de dominance étant agencé pour classer l'ensemble de signatures (1, 2) en fonction d'au moins un critère de performance, et calculer une valeur de dominance pour chaque signature, la valeur de dominance étant le nombre de signatures avec un classement supérieur pour lesdits critères de performance, et fixer un seuil de dominance et supprimer les signatures ayant une valeur de dominance supérieure audit seuil de dominance.

**15.** Produit de programme informatique comprenant un code informatique pouvant être exécuté par des moyens de traitement afin d'effectuer le procédé de l'une des revendications 1 à 13 lorsque le code informatique est exécuté.

Fig. 1

| Colon cancer | | | |
|---|---|---|---|
| Filter | Number of signature before the filter | Number of signature after the filter | Minimum number of biomarker |
| First performance (3,4) | 2000 | 123 | 210 |
| First frequency (5,6) | 123 | 119 | 97 |
| First dominance (7,8) | 123 | 46 | 20 |
| Intersection first frequency (5,6) and first dominance (7,8) | 22 | | 17 |
| Second dominance (9,10) | 22 | 22 | 17 |
| Second performance (11,12) | 22 | 8 | 10 |
| Third performance (13,14) | 8 | 1 | 10 |
| Expert (15, 16) | 1 | 1 | 2 |

Fig. 2

| Colon cancer | | |
|---|---|---|
| Technique | Accuracy (%) | Number of biomarker in the signature |
| **Present invention** | **94.14** | **2** |
| TSP | 91.10 | 2 |
| k-TSP | 90.30 | 2 |
| Fuzzy logic | 90.00 | 17 |
| PAM | 85.48 | 15 |
| DT | 80.65 | 3 |

Fig. 3

| Prostate cancer | | | |
|---|---|---|---|
| Filter | Number of signature before the filter | Number of signature after the filter | Minimum number of biomarker |
| First performance (3,4) | 900 | 31 | 148 |
| First frequency (5,6) | 31 | 31 | 127 |
| First dominance (7,8) | 31 | 25 | 148 |
| Intersection first frequency (5,6) and first dominance (7,8) | 25 | | 127 |
| Second dominance (9,10) | 25 | 25 | 127 |
| Second performance (11,12) | 25 | 8 | 5 |
| Third performance (13,14) | 8 | 1 | 5 |
| Expert (15, 16) | 1 | 1 | 2 |

Fig. 4

| Prostate cancer | | |
|---|---|---|
| Technique | Accuracy (%) | Number of biomarker in the signature |
| **Present invention** | **97.29** | **2** |
| TSP | 95.00 | 2 |
| k-TSP | 91.00 | 2 |
| PAM | 91.00 | 47 |
| DT | 80.65 | 4 |

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013190086 A
- EP 0827611 A
- US 2012115138 A
- US 2012040861 A

**Non-patent literature cited in the description**

- **RESSOM HABTOM.** Classification algorithms for phenotype prediction in genomics and proteomics. *FRONTIERS IN BIOSCIENCE,* 2008, vol. 13 (13), 691
- **CARLOS ANDRES.** Coevolutionary fuzzy modelling. *PENA-REYES,* 2002
- **ALON, U. ; BARKAI, N. ; NOTTERMAN, D. A. ; GISH, K. ; YBARRA, S. ; MACK, D. ; LEVINE, A. J.** Broad patterns of gene expression revealed by clustering analysis of tumor and normal colon tissues probed by oligonucleotide arrays. *Proceedings of the National Academy of Sciences,* 1999, vol. 96 (12), 6745-6750
- **SINGH, D. ; FEBBO, P. G. ; ROSS, K. ; JACKSON, D. G. ; MANOLA, J. ; LADD, C. ; SELLERS, W. R.** Gene expression correlates of clinical prostate cancer behavior. *Cancer cell,* 2002, vol. 1 (2), 203-209